Europäisches Patentamt

European Patent Office

Office européen des brevets

(19)

(11) Publication number: **0 044 740**
**B1**

## (12) EUROPEAN PATENT SPECIFICATION

(45) Date of publication of patent specification: **09.05.84**

(21) Application number: **81303326.3**

(22) Date of filing: **21.07.81**

(51) Int. Cl.³: **C 07 C 1/04, C 07 C 29/15, B 01 J 21/08, B 01 J 23/74, B 01 J 23/80, B 01 J 23/84, B 01 J 27/24**

(54) Process for the production of lower hydrocarbons and oxygenated derivatives thereof by the catalytic conversion of carbon monoxide and hydrogen.

(30) Priority: **23.07.80 GB 8024077**

(43) Date of publication of application:
**27.01.82 Bulletin 82/4**

(45) Publication of the grant of the patent:
**09.05.84 Bulletin 84/19**

(84) Designated Contracting States:
**BE DE FR GB IT NL**

(56) References cited:
**DE - A - 2 623 867**
**DE - A - 2 818 307**
**DE - A - 2 822 656**
**DE - A - 2 925 571**
**US - A - 4 086 261**
**US - A - 4 142 061**
**US - A - 4 168 245**

(73) Proprietor: **The British Petroleum Company p.l.c.**
**Britannic House Moor Lane**
**London EC2Y 9BU (GB)**

(72) Inventor: **Ball, William John**
**Chertsey Road**
**Sunbury-on-Thames Middlesex, TW16 7LN (GB)**
Inventor: **Stewart, David Gordon**
**Chertsey Road**
**Sunbury-on-Thames Middlesex, TW16 7LN (GB)**

(74) Representative: **Harry, John et al,**
**BP INTERNATIONAL LIMITED Patents and**
**Licensing Division Chertsey Road**
**Sunbury-on-Thames Middlesex TW16 7LN (GB)**

Courier Press, Leamington Spa, England.

## Description

The present invention relates to a process for the production of $C_1$ to $C_4$ hydrocarbons and oxygenated derivatives thereof by the catalytic conversion of carbon monoxide and hydrogen, hereinafter to be referred to as synthesis gas.

During the last few years political and economic events affecting the petroleum industry have led to increasing interest in alternatives to petroleum as the practically exclusive source of raw materials for the petrochemical industry. In this search for alternatives there has been a resurgence of interest in the long and universally known Fisher/Tropsch process whereby hydrocarbons and oxygen-containing compounds are obtained from carbon monoxide and hydrogen which can be obtained from other carbonaceous deposits such as coal and natural gas. The raw materials demanded by the petrochemical industry are hydrocarbons and in particular unsaturated hydrocarbons with a chain length from $C_2$ to $C_4$. Unfortunately, the products obtained from the Fisher/Tropsch process using the cobalt and iron catalysts conventionally employed are mainly liquid and partailly solid hydrocarbons having chain lengths from $C_6$ to about $C_{50}$, oxygenated hydrocarbons and, at the most, only 30 to 50% by weight of the low molecular weight hydrocarbons of chain length $C_2$ to $C_4$ which are desirable in the petrochemical industry.

One catalyst which has been investigated in other types of reaction is silica based material comprising crystalline silica which has been modified with one or more elements which have entered the crystalline lattice of the silica in place of silicon atoms of the silica or in the form of salts of bisilicic or polysilicic acids. Such catalysts are described in published U.K. Patent Specification No. 2,024,790A. Preferred elements are said to be those having, at least partially, an amphoteric character such as chromium, beryllium, titanium, vanadium, manganese, iron, cobalt, zinc, zirconium, rhodium, silver, tin, antimony and boron, of which the use of beryllium, chromium, zinc, titanium, vanadium and boron are exemplified. The crystalline materials are described as catalysts for a large number of reactions, including the conversion of dimethyl ether and/or methanol or other lower alcohols into hydrocarbons such as olefins. However, the list of catalytic applications does not include the production of hydrocarbons by the catalytic conversion of carbon monoxide and hydrogen.

During the course of our investigations we produced a crystalline silica modified with cobalt by mixing a source of silica, a source of cobalt, a nitrogenous base and a mineralising agent in water and maintaining the mixture under elevated temperature and pressure for a considerable period of time. After calcination the cobalt modified silica, instead of converting methanol into hydrocarbons such as ethylene, quantitatively decomposed the methanol to synthesis gas. This reaction forms the subject of our European Patent Publication No. 38682. To our surprise, the reverse reaction, i.e. the catalytic hydrogenation of carbon monoxide, produced only a small proportion of $C_1$ to $C_4$ oxygenated hydrocarbons which consisted mainly of methanol, but included also ethanol, and a major proportion of $C_1$ to $C_4$ hydrocarbons and substantially no hydrocarbons of higher carbon number.

Accordingly, the present invention provides a process for the production of $C_1$ to $C_4$ hydrocarbons and oxygenated derivatives thereof by contacting synthesis gas at a temperature in the range 150 to 450°C and a pressure in the range 1 to 700 bars with a catalyst characterised in that the catalyst is the crystalline cobalt-modified silica product obtained by mixing a source of silica, a source of cobalt and a nitrogenous base in a liquid medium comprising either water, an alcohol or a mixture thereof and maintaining the mixture under conditions of elevated temperature and pressure for a time sufficient to effect crystallisation of the product.

Mixtures of the gases hydrogen and carbon monoxide are abundantly available in the form of synthesis gas. Methods for preparing synthesis gas are well known in the art and usually involve the partial oxidation of a carbonaceous substance, e.g. coal. Alternatively, synthesis gas may be prepared, for example, by the catalytic steam reforming of methane. Although it is preferred to use substantially pure synthesis gas, the presence of such impurities as carbon dioxide and nitrogen can be tolerated. On the other hand, impurities which have a deleterious effect on the reaction should be avoided. The ratio of hydrogen to carbon monoxide in the synthesis gas may vary widely. Normally the molar ratio of hydrogen to carbon monoxide may be in the range from 5:1 to 1:5, preferably from 2:1 to 1:2. In general a high proportion of hydrogen favours the formation of hydrocarbons whilst a low proportion of hydrogen favours the formation of oxygenated hydrocarbons. Methods for adjusting the molar ratio of hydrogen to carbon monoxide by the so-called shift reaction are well known in the art.

Preferably the temperature is in the range from 200 to 400°C, even more preferably from 200 to 300°C and the pressure is in the range from 10 to 300 bars. The use of higher temperature within the aforesaid ranges tend to increase the co-production of methane. Because of the highly exothermic nature of the reaction the temperature requires careful control in order to prevent a runaway methanation, in which methane formation increases with increasing temperature and the resulting exotherm increases the temperature still further. In fixed bed operations, temperature control may be achieved by mixing the catalyst with an inert diluent, thereby ensuring that the exothermic heat is more evenly distributed. In this way the useful life of the catalyst may be prolonged. The use of higher pressures within the aforesaid ranges tend to increase the production rate and selectivity to $C_1$ to $C_4$ hydrocarbons.

Although the process may be carried out batchwise it is preferably operated in a continuous manner. Suitably the contact time, as hereinafter defined, for continuous operation may be up to 30, preferably from 0.01 to 5 seconds. For the purpose of this specification the contact time is defined as:

Volume of catalyst in millilitres

Total volume of gas (in millilitres/second at NTP)

The catalyst may be employed in the form of a fixed or a fluidised bed.

The catalyst is the crystalline product obtained by mixing a source of silica, a source of cobalt and a nitrogenous base in a liquid medium comprising water, an alcohol or a mixture thereof and maintaining the mixture under conditions of elevated temperature and pressure for a time sufficient to effect crystallisation of the mixture.

Suitable sources of silica include, for example, sodium silicate, silica hydrosol, silica gel, silica sol and silicic acid. The preferred source of silica is an aqueous colloidal dispersion of silica particles. A suitable commercially available source of silica is LUDOX Colloidal Silica marketed by Du Pont (LUDOX is a Registered Trade Mark).

Suitable sources of cobalt include the oxide, hydroxide, salts such as the nitrate and alkoxy derivatives such as the acetate.

Suitable sources of nitrogenous base include amines and substituted amines such as the alkanolamines. A preferred nitrogenous base is a quaternary ammonium compound, e.g. a tetraalkylammonium compound in which the alkyl group contains from 1 to 5 carbon atoms or a tetraarylammonium compound in which the aryl group is a phenyl or an alkylphenyl group.

Optionally, a mineralising agent may be employed. Suitable mineralising agents include alkali metal and alkaline earth metal hydroxides and halides, for example LiOH, NaOH, $Ca(OH)_2$, KBr, NaBr, NaI, $CaI_2$ and $CaBr_2$.

Optionally, an inorganic base may be employed. Suitable inorganic bases include alkali metal and alkaline earth metal hydroxides, for example NaOH, KOH and $Ca(OH)_2$, and ammonia.

The liquid medium may additionally contain an active organic species such as an alkyl halide, e.g. methyl bromide, ethyl iodide or propyl bromide.

The amount of nitrogenous base and/or inorganic base employed may vary within wide limits.

The mixture may suitably be maintained at a temperature of from 100 to 200, preferably from 120 to 200°C for a period of from a few hours to several days, preferably for about 3 days. The mixture is preferably maintained at the required temperature in a closed vessel such as an autoclave under autogenous pressure.

The modified crystalline silica may suitably be separated from the liquid medium, after cooling, by filtration. After separation the silica is preferably washed to remove any exchangeable cationic impurities, preferably with boiling distilled water having dissolved therein an ammonium salt such as the nitrate or acetate.

The crystalline product obtained is believed to be a crystalline silica modified by inclusion of cobalt in the crystal lattice in place of a proportion of the silicon atoms. It is preferred to calcine the crystalline product before use as a catalyst. Calcination may be effected by heating in air at a temperature in the range from 300 to 700°C, preferably at about 550°C, for a period of from 2 to 24 hours.

The catalytic activity of the crystalline product may be further enhanced by the addition of one and more metals. Suitable metals which may be added include cobalt, iron, manganese, nickel, ruthenium, rhodium, palladium, zinc, chromium and copper. The metals may be added either by impregnation or by ion-exchange. Any of the known techniques for impregnation and ion-exchange may be employed. The crystalline product may suitably contain from 0.1 to 20%, preferably from 0.2 to 10% by weight of added metal.

The invention will now be particularly described by reference to the following Examples and by reference to the accompaning Figure which is a simplified diagram of the apparatus employed.

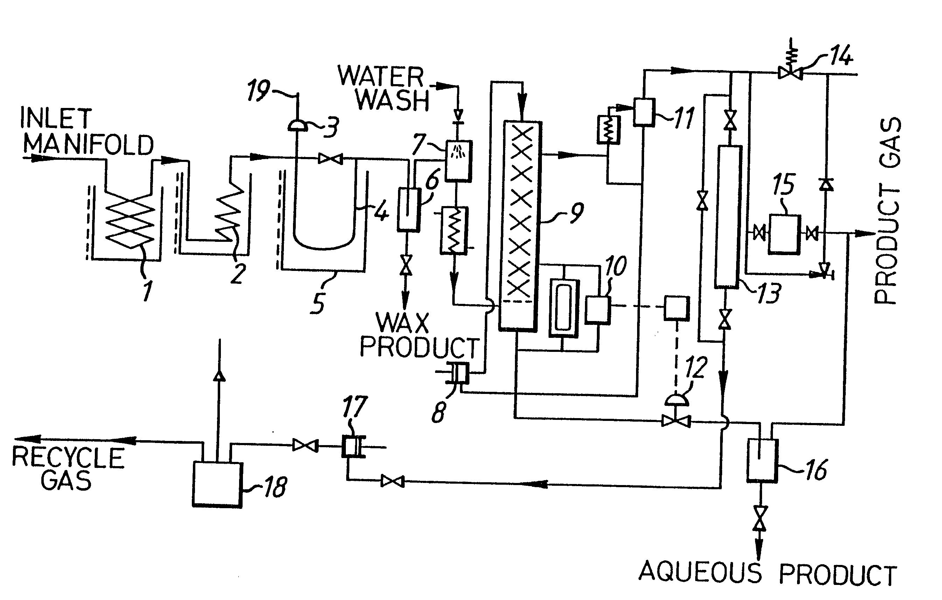

With reference to the Figure, 1 is a preheater (150°C), 2 is a preheater (200°C), 3 is a bursting disc, 4 is a reactor, 5 is a salt pot, 6 is a knock-out pot, 7 is a water quench, 8 is a water recycle pump, 9 is a water wash tower, 10 is a differential pressure level controller, 11 is a knock-out pot, 12 is a Foxboro valve, 13 is a molecular sieve drier, 14 is a Gyp relief valve, 15 is a back pressure regulator, 16 is an aqueous product receiver, 17 is a gas recycle pump, 18 is a ballast vessel and 19 is a vent.

## Catalyst Preparations

### Catalyst A
#### (cobalt silicate)

Sodium nitrate (0.85 g) was dissolved in aqueous tetrapropyl ammonium hydroxide solution and then cobalt nitrate hexahydrate (2.9 g) was added. A blue solid was formed. Ludox silica sol, Type AS40 (21.8 g, containing 40% silica), was added with vigorous stirring at room temperature and the suspension was allowed to stand for two hours.

The mixture was placed in a pressure vessel which was rotated and heated at 150°C for 24 hours and then at 170°C for 60 hours. At this point the mixture was cooled to room temperature, filtered and the solid washed by heating with a 1 molar ammonium chloride solution (150 ml). This operation was repeated twice. Finally the solid was washed with deionised water (300 ml), dried at 120°C for 16 hours and broken down to form 8—16 mesh granules. Analysis of the solid showed Si 40.5 and Co 6.06% wt/wt.

### Catalyst B
### (cobalt treated cobalt silicate)

Cobalt nitrate hexahydrate (7 g) was dissolved in deionised water (500 ml) and the resulting solution was divided into three portions.

Catalyst A (14 g) was suspended in one portion of the above solution and the mixture was heated at 80°C for 90 minutes with stirring. This operation was carried out three times. The final solid was washed with deionised water (300 ml), dried at 120°C, calcined in air at 500°C for 16 hours and broken down to form 8—16 mesh (B.S.S.) granules. Analysis of the product showed silicon (40.3 and cobalt 9.6% wt/wt.

### Catalyst C
### (rhodium treated cobalt silicate)

Rhodium trichloride trihydrate (1.3 g) was dissolved in deionised water (500 ml) and the resulting solution was divided into three portions.

Catalyst A (14 g) was suspended in one portion of the above solution and the mixture was heated at 80°C for 90 minutes with stirring. This operation was carried out three times. The final solid was washed with deionised water (300 ml), dried at 120°C, calcined in air at 500°C for 16 hours and broken down to pass 8—16 mesh (B.S.S.) granules. Analysis of the product showed silicon 38.0, cobalt 9.9 and rhodium 2.9% wt/wt.

### Catalyst D
### (rhodium treated cobalt silicate)

Catalyst A (14 g) was suspended in a solution of rhodium trichloride trihydrate (1.3 g) in deionised water (20 ml). The mixture was evaporated to dryness on a steam bath and the solid dried at 120°C and broken down to form 8—16 mesh (B.S.S.) granules.

### Catalyst Testing

The catalysts were reduced in hydrogen at 450°C for 6 hours and were tested for the conversion of carbon monoxide/hydrogen mixtures.

### Example 1

With reference to the accompanying Figure a mixture of carbon monoxide and hydrogen in a molar ratio of 1:2 was passed via the inlet manifold through the two preheater coils (1) and (2) maintained at 150°C and 200°C respectively in silicone oil baths. The heated gases were then fed via a heat-traced line to the copper-lined reactor (4) containing a fixed bed of Catalyst A in the form of 8 to 16 mesh (B.S.S.) granules. The reactor was maintained at the desired reaction temperature by immersion in a molten salt bath (5). The product gases were passed via a heat-traced line through a knock-out pot for wax products (6) to a small quench vessel (7) into the top of which water was sprayed. The gases were then passed through a water cooler to the bottom of the water wash tower (9) which was packed with 3/8 inch Raschig rings. In the tower (9) the product gases were washed counter-current with water. The resulting liquid product was fed into the receiver (16) and any dissolved gases were recombined with the product gas stream from the back pressure regulator (15). The separated gas stream from the top of the water wash tower (9) was passed through a water cooler to the knock-out pot (11) and then to the inlet side of the dome-loaded back pressure regulator (15).

Recycle gas was recovered from the inlet side of the back pressure regulator (15), passed through a molecular sieve drier (13) and compressed up to 67 bars in the gas ballast vessel (18) using the gas recycle pump (17). The recycle gas was fed back to the inlet manifold. Provision was made to feed spot samples of the inlet gases and the total gas stream to a gas chromatographic analytical unit.

The product gas stream leaving the back pressure regulator (15) was measured and samples were withdrawn and analysed by gas chromatography. The liquid product was also sampled and analysed by gas chromatography.

After the reaction had reached equilibrium balance runs were carried out over one hour periods.

### Example 2

The procedure of Example 1 was repeated except that Catalyst A was replaced by Catalyst B.

### Example 3

The procedure of Example 1 was repeated except that Catalyst A was replaced by Catalyst C.

### Example 4

The procedure of Example 1 was repeated except that Catalyst A was replaced by Catalyst D.

The reaction conditions and results for Examples 1 to 4 are given in the accompanying Table.

TABLE

Reaction parameters:  GHSV = 48,000 h$^{-1}$
H$_2$:CO molar ratio = 2:1
Pressure = 50 bars
Recycle gas ratio = 19:1

| Example | Catalyst | Reaction Temperature °C | CO Conversion[1] % | Selectivity[2] (%) | | | | | |
|---|---|---|---|---|---|---|---|---|---|
| | | | | CO$_2$ | CH$_4$ | C$_2$H$_6$ | C$_3$H$_8$ | Methanol | Ethanol |
| 1 | A | 276 | 13 | 2 | 61 | 7 | 11 | 11 | 8 |
| | | 313 | 22 | 2 | 60 | 9 | 9 | 15 | 5 |
| 2 | B | 277 | 9 | 9 | 48 | 7 | 11 | 15 | 10 |
| | | 313 | 19 | 11 | 58 | 8 | 8 | 9 | 6 |
| 3 | C | 283 | 10 | 24 | 60 | 4 | 4 | 5 | 3 |
| | | 350 | 20 | 22 | 48 | 6 | 5 | 10 | 9 |
| 4 | D | 369 | 4 | 12 | 57 | 4 | — | 21 | 6 |

1. CO Conversion $= \dfrac{\text{Moles of carbon monoxide consumed}}{\text{Moles of carbon monoxide fed}} \times 100$

2. Selectivity $= \dfrac{\text{Moles of carbon monoxide converted to particular product}}{\text{Moles of carbon monoxide consumed}} \times 100$

**Claims**

1. A process for the production of $C_1$ to $C_4$ hydrocarbons and oxygenated derivatives thereof by contacting synthesis gas at a temperature in the range 150 to 450°C and a pressure in the range 1 to 700 bars with a catalyst characterised in that the catalyst is the crystalline cobalt-modified silica product obtained by mixing a source of silica, a source of cobalt and a nitrogenous base in a liquid medium comprising either water, an alcohol or a mixture thereof and maintaining the mixture under conditions of elevated temperature and pressure for a time sufficient to effect crystallisation of the product.

2. A process according to claim 1 wherein the temperature is in the range 200 to 300°C.

3. A process according to either claim 1 or claim 2 wherein the pressure is in the range 10 to 300 bars.

4. A process according to any one of the preceding claims when operated in a continuous manner at a contact time of up to 30 seconds.

5. A process according to claim 4 wherein the contact time is in the range from 0.01 to 5 seconds.

6. A process according to any one of the preceding claims wherein the nitrogenous base employed in the preparation of the cobalt-modified silica product is a tetraalkylammonium compound in which the alkyl group contains from 1 to 5 carbon atoms or a tetraarylammonium compound in which the aryl group is a phenyl or an alkylphenyl group.

7. A process according to any one of the preceding claims wherein a mineralising agent is employed in the preparation of the cobalt-modified silica product.

8. A process according to any one of the preceding claims wherein an inorganic base is employed in the preparation of the cobalt-modified silica product.

9. A process according to any one of the preceding claims wherein the crystalline cobalt-modified silica is calcined by heating in air at a temperature in the range from 300 to 700°C for a period of from 2 to 24 hours prior to contact with the synthesis gas.

10. A process according to any one of the preceding claims wherein there is added to the crystalline cobalt-modified silica one or more of the metals cobalt, iron, manganese, nickel, ruthenium, rhodium, palladium, zinc, chromium and copper.

**Revendications**

1. Procédé de production d'hydrocarbures en $C_1$ à $C_4$ et de leurs dérivés oxygénés, par mise en contact du gaz de synthèse, à une température comprise entre 150 et 450°C et à une pression comprise entre 1 et 700 bars, avec un catalyseur, procédé caractérisé en ce que le catalyseur est le produit, de type silice crystalline modifiée par du cobalt, que l'on obtient en mélangeant une source de silice, une source de cobalt et une base azotée dans un milieu liquide comprenant de l'eau, un alcool ou un de leurs mélanges, et en maintenant le mélange dans des conditions de température et de pression élevées pendant un temps suffisant pour réaliser la cristallisation du produit.

2. Procédé selon la revendication 1, dans lequel la température se situe entre 200 et 300°C.

3. Procédé selon la revendication 1 ou la revendication 2, dans lequel la pression se situe entre 10 et 300 bars.

4. Procédé selon l'une quelconque des revendications précédentes, lorsqu'il est mis en oeuvre de façon continue avec un temps de contact pouvant aller jusqu'à 30 secondes.

5. Procédé selon la revendication 4, dans lequel le temps de contact se situe entre 0,01 et 5 secondes.

6. Procédé selon l'une quelconque des revendications précédentes, dans lequel la base azotée utilisée dans la préparation du produit de type silice modifiée par du cobalt est un composé de tétraalkylammonium dans lequel le groupe alkyle contient 1 à 5 atomes de carbone ou est un composé de tétraarylammonium dans lequel le groupe aryle est un groupe phényle ou un groupe alkylphényle.

7. Procédé selon l'une quelconque des revendications précédentes, dans lequel on utilise un agent de minéralisation en présence du produit de type silice modifiée par du cobalt.

8. Procédé selon l'une quelconque des revendications précédentes, dans lequel on utilise une base minérale pour la préparation du produit de type silice modifiée par du cobalt.

9. Procédé selon l'une quelconque des revendications précédentes, dans lequel on calcine la silice cristalline modifiée par du cobalt en la chauffant dans de l'air à une température comprise entre 300 et 700°C pendant une période de 2 à 24 heures avant de la mettre en contact avec le gaz de synthèse.

10. Procédé selon l'une quelconque des revendications précédentes, dans lequel on ajoute à la silice cristalline modifiée par du cobalt un ou plusieurs des métaux cobalt, fer, manganèse, nickel, ruthénium, rhodium, palladium, zinc, chrome et cuivre.

**Patentansprüche**

1. Verfahren zur Herstellung von $C_1$—$C_4$-Kohlenwasserstoffen und oxygenierten Derivaten davon durch Kontaktieren von Synthesegas bei einer Temperatur im Bereich von 150 bis 450°C und einem Druck im Bereich von 1 bis 700 bar mit einem Katalysator, dadurch gekennzeichnet, daß der Katalysator ein kristallines, kobalt-modifiziertes Siliciumdioxidprodukt ist, das erhalten wurde durch Vermischen einer Siliciumdioxidquelle, einer Kobaltquelle und einer Stickstoffbase in einem flüssigen Medium, das entweder Wasser, einen Alkohol oder ein Gemisch davon umfaßt, und Halten des Gemisches bei erhöhten Temperatur- und Druckbedingungen über ausreichende Zeit, um eine Kristallisation des Produkts zu bewirken.

2. Verfahren nach Anspruch 1, worin die Temperatur im Bereich von 200 bis 300°C liegt.

3. Verfahren nach entweder Anspruch 1 oder Anspruch 2, worin der Druck im Bereich von 10 bis 300 bar liegt.

4. Verfahren nach irgendeinem der vorangehenden Ansprüche, das kontinuierlich mit einer Kontaktzeit von bis zu 30 Sekunden durchgeführt wird.

5. Verfahren nach Anspruch 4, worin die Kontaktzeit im Bereich von 0,01 bis 5 Sekunden liegt.

6. Verfahren nach iregendeinem der vorangehenden Ansprüche, worin die zur Herstellung des kobalt-modifizierten Siliciumdioxidprodukts verwendete Stickstoffbase eine Tetralkylammoniumverbindung, in der die Alkylgruppe 1 bis 5 Kohlenstoffatome enthält, oder einer Tetraarylammoniumverbindung ist, in der die Arylgruppe eine Phenyl- oder Alkylphenylgruppe ist.

7. Verfahren nach irgendeinem der vorangehenden Ansprüche, worin bei der Herstellung des kobalt-modifizierten Siliciumdioxidprodukts ein Mineralisierungsmittel verwendet wird.

8. Verfahren nach irgendeinem der vorangehenden Ansprüche, worin bei der Herstellung des kobalt-modifizierten Silicium-dioxidprodukts eine anorganische Base verwendet wird.

9. Verfahren nach irgendeinem der vorangehenden Ansprüche, worin das kristalline, kobalt-modifizierte Siliciumdioxid vor dem Kontakt mit dem Synthesegas calciniert wird durch Erhitzen an der Luft bei einer Temperatur im Bereich von 300 bis 700°C über eine Zeitspanne von 2 bis 24 Stunden.

10. Verfahren nach irgendeinem der vorangehenden Ansprüche, worin das kristalline, kobalt-modifizierte Siliciumdioxid mit einem oder mehreren der Metalle Kobalt, Eisen, Mangan, Nickel, Ruthenium, Rhodium, Palladium, Zink, Chrom und Kupfer versetzt wird.

PRODUCT GAS

AQUEOUS PRODUCT

WATER WASH

WAX PRODUCT

INLET MANIFOLD

RECYCLE GAS